(19) Europäisches Patentamt
European Patent Office
Office européen des brevets

(11) Veröffentlichungsnummer: **0 339 417 B1**

# (12) EUROPÄISCHE PATENTSCHRIFT

(45) Veröffentlichungstag der Patentschrift:
**11.09.91 Patentblatt 91/37**

(51) Int. Cl.[5]: **C07D 207/34,** A01N 43/36

(21) Anmeldenummer: **89106817.3**

(22) Anmeldetag: **17.04.89**

## (54) 3-Cyano-4-phenyl-pyrrole.

Die Akte enthält technische Angaben, die nach dem Eingang der Anmeldung eingereicht wurden und die nicht in dieser Patentschrift enthalten sind.

(30) Priorität: **29.04.88 DE 3814478**

(43) Veröffentlichungstag der Anmeldung:
**02.11.89 Patentblatt 89/44**

(45) Bekanntmachung des Hinweises auf die Patenterteilung:
**11.09.91 Patentblatt 91/37**

(84) Benannte Vertragsstaaten:
**BE CH DE ES FR GB IT LI NL**

(56) Entgegenhaltungen:
**EP-A- 0 130 149**
**EP-A- 0 174 910**
**EP-A- 0 236 272**
**DE-A- 2 927 480**
**CA 95 No.21 S. 637 (187069f)**

(73) Patentinhaber: **BAYER AG**
**W-5090 Leverkusen 1 Bayerwerk (DE)**

(72) Erfinder: **Wollweber, Detlef, Dr.**
**Paul-Ehrlich-Strasse 17**
**W-5600 Wuppertal 1 (DE)**
Erfinder: **Brandes, Wilhelm, Dr.**
**Eichendorffstrasse 3**
**W-5653 Leichlingen (DE)**
Erfinder: **Dutzmann, Stefan, Dr.**
**Leinenweberweg 33**
**W-4000 Duesseldorf 13 (DE)**

**EP 0 339 417 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 3-Cyano-4-phenyl-pyrrole, ein Verfahren zu ihrer Herstellung und ihre Verwendung in Schädlingsbekämpfungsmitteln.

Es ist bekannt, daß bestimmte 3-Cyano-4-phenyl-pyrrole,wie beispielsweise die Verbindung 3-Cyano-4-(2,3-di-chlorphenyl)-pyrrol oder die Verbindung 1-Acetyl-3-cyano-4-(2,3-dichlorphenyl)-pyrrol, fungizide Wirksamkeit besitzen (vgl. z.B. EP 236272 ; DE-OS 2927480).

Weiterhin sind 3-Cyano-4-phenyl-pyrrole, wie beispielsweise die Verbindung N-Methoxymethylcarbonyl-3-cyano-4(2,3-dichlorphenyl)-pyrrol, bekannt, die eine fungizide Wirksamkeit besitzen (vgl. Jp. Kokai Tokkyo Koho 8179, 672) oder N-[1-(Methoxyacetyloxy)-2,2,2-trichlorethyl]-3-(2,3-dichlorphenyl)-4-cyano-pyrrol zum Beispiel mit fungizider Wirkung (vgl. EP 130149). Auch sind neue Verfahren zur Herstellung von 4-Phenyl-pyrrol-Derivaten bekannt (vgl. EP 174910).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue 3-Cyano-4-phenyl-pyrrole der allgemeinen Formel (I),

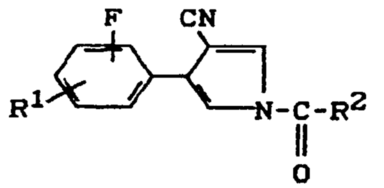

(I)

in welcher

$R^1$ für Chlor steht und

$R^2$ für jeweils geradkettiges oder verzweigtes Alkyl, Alkoxy oder Alkoxyalkyl mit jeweils 1 bis 6 Kohlenstoffatomen in den einzelnen Alkylteilen steht,

gefunden.

Weiterhin wurde gefunden, daß man die neuen 3-Cyano-4-phenyl-pyrrole der allgemeinen Formel (I),

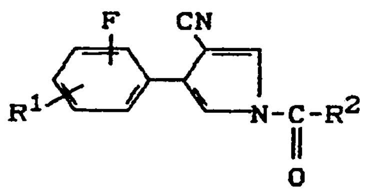

(I))

in welcher

$R^1$ für Chlor steht und

$R^2$ für jeweils geradkettiges oder verzweigtes Alkyl, Alkoxy oder Alkoxyalkyl mit jeweils 1 bis 6 Kohlenstoffatomen in den einzelnen Alkylteilen steht,

erhält, wenn man 1H-3-Cyano-4-phenyl-pyrrole der Formel (II),

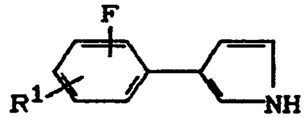

(II)

in welcher

$R^1$ die oben angegebene Bedeutung hat,

mit Acylierungsmitteln der Formel (III),

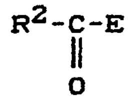

(III)

in welcher

$R^2$ die oben angegebene Bedeutung hat und

E für eine elektronenanziehende Abgangsgruppe steht, gegebenenfalls in Gegenwart eines Verdünnungsmittels und gegebenenfalls in Gegenwart eines Reaktionshilfsmittels umsetzt.

Schließlich wurde gefunden, daß die neuen 3-Cyano-4-phenyl-pyrrole der allgemeinen Formel (I) eine gute Wirkung gegen Schädlinge besitzen.

Überraschenderweise zeigen die erfindungsgemäßen 3-Cyano-4-phenyl-pyrrole der allgemeinen Formel (I) u.a. eine erheblich bessere fungizide Wirksamkeit als die aus dem Stand der Technik bekannten 3-Cyano-4-phenyl-pyrrole, wie beispielsweise das 3-Cyano-4-(2,3-dichlorphenyl)-pyrrol, welche chemisch und wirkungsmäßig naheliegende Verbindungen sind.

Die erfindungsgemäßen 3-Cyano-4-phenyl-pyrrole sind durch die Formel (I) allgemein definiert. Bevorzugt sind Verbindungen der Formel (I), bei welchen

$R^1$ für Chlor steht und

$R^2$ für jeweils geradkettiges oder verzweigtes Alkyl, Alkoxy oder Alkoxyalkyl mit jeweils 1 bis 4 Kohlenstoffatomen in den einzelnen Alkylteilen steht.

Ganz besonders bevorzugt sind Verbindungen der Formel (I), bei welchen

$R^1$ für Chlor steht und

$R^2$ für Methyl, Ethyl, Methoxy, Ethoxy, Methoxymethyl oder Ethoxymethyl steht.

Im einzelnen seien außer den bei den Herstellungsbeispielen genannten Verbindungen die folgenden 3-Cyano-4-phenyl-pyrrole der allgemeinen Formel (I) genannt:

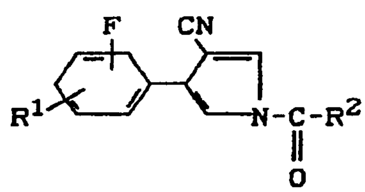

(I)

| 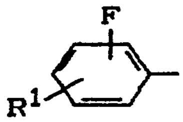 | $R^2$ | 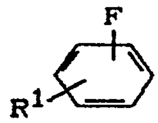 | $R^2$ |
|---|---|---|---|
| 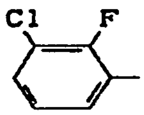 | $-C_2H_5$ | 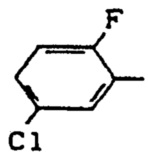 | $-CH_3$ |
| 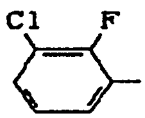 | $-CH_2-OC_2H_5$ | 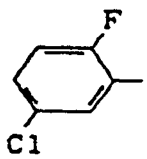 | $-OCH_3$ |
| 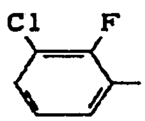 | $-(CH_2)_2-CH_3$ | 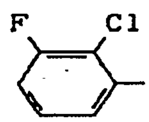 | $-CH_3$ |

| 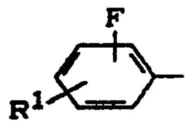 | $R^2$ | 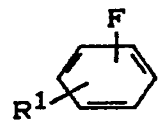 | $R^2$ |
|---|---|---|---|
| 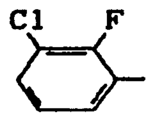 | $-(CH_2)_3-CH_3$ | 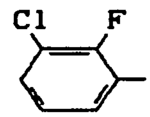 | $-CH_2-OCH_3$ |
| 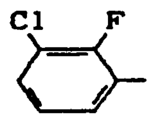 | $-CH_2-O-(CH_2)_2-CH_3$ | 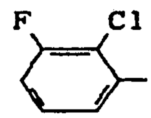 | $-C_2H_5$ |
| 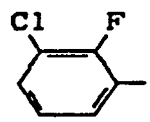 | $-CH(CH_3)_2$ | 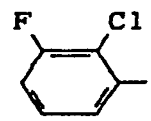 | $-OCH_3$ |
| 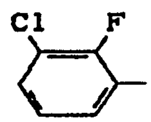 | $-OC_2H_5$ | 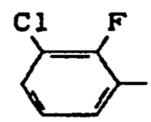 | $-OC_2H_5$ |
| 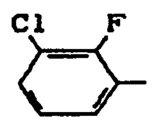 | $-O-(CH_2)_2-CH_3$ | 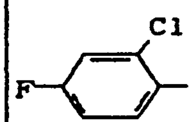 | $-CH_3$ |
| 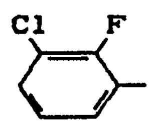 | $-O-CH(CH_3)_2$ | 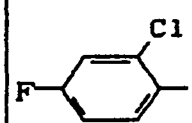 | $-CH_2-OCH_3$ |
| 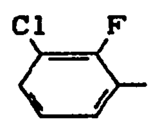 | $-O-CH(CH_3)-C_2H_5$ | 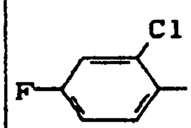 | $-OCH_3$ |
| 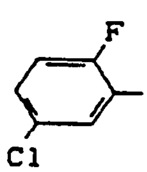 | $-OC_2H_5$ | 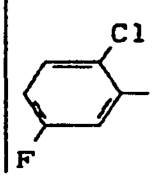 | $-CH_3$ |

| 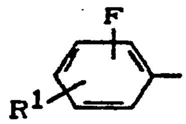 | $R^2$ | 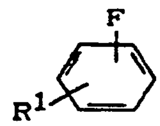 | $R^2$ |
|---|---|---|---|
| | | 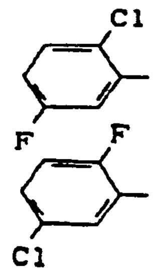 | $-CH_2-OCH_3$ |
| 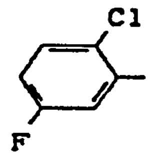 | $-OCH_3$ | | $-C_2H_5$ |
| | | 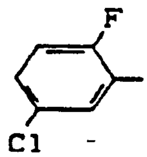 | $-CH_2-OCH_3$ |

Verwendet man beispielsweise 3-Cyano-4-(2-fluor-3-chlorphenyl)-pyrrol und Chlorameisensäureethylester als Ausgangsstoffe, so läßt sich der Reaktionsablauf des erfindungsgemäßen Verfahrens durch das folgende Formelschema darstellen :

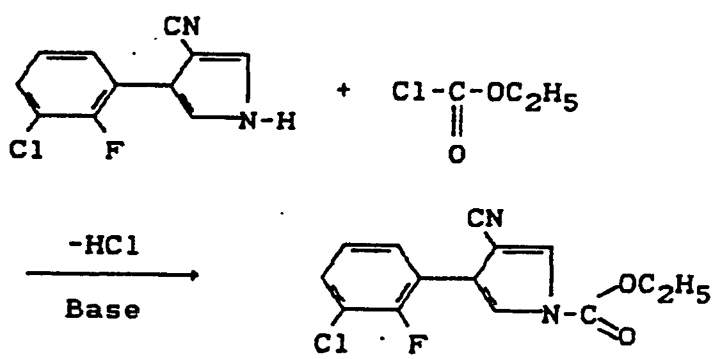


Die zur Durchführung des erfindungsgemäßen Verfahrens als Ausgangsstoffe benötigten 1H-3-Cyano-4-phenyl-pyrrole sind durch die Formel (II) allgemein definiert. In dieser Formel (II) steht Ar vorzugsweise für diejenigen Reste, die bereits im Zusammenhang mit der Beschreibung der erfindungsgemäßen Stoffe der Formel (I) als bevorzugt für diesen Substituenten genannt wurden.

Die 1H-3-Cyano-4-phenyl-pyrrole der Formel (II) sind noch nicht bekannt. Sie sind jedoch Gegenstand der eigenen vorgängigen, noch nicht publizierten deutschen Patentanmeldung P 3737984 vom 09.11.1987 und erhältlich in Analogie zu bekannten Verfahren (vgl. z.B. EP 236272, DE-OS 2927480), beispielsweise wenn man

a) Fluoraniline der Formel (IV),

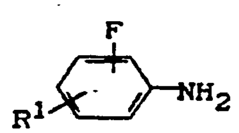 (IV)

in welcher

$R^1$ die oben angegebene Bedeutung hat,

zunächst in einer ersten Stufe mit Acrylnitril unter üblichen Diazotierungsbedingungen, beispielsweise in Gegenwart von Natriumnitrit und Salzsäure, und in Gegenwart eines geeigneten Metallsalz-Katalysators, wie

beispielsweise Kupfer-II-chlorid oder Kupfer-II-oxid, und gegebenenfalls in Gegenwart eines geeigneten Verdünnungsmittels, wie beispielsweise Aceton oder Wasser, bei Temperaturen zwischen −20°C und +50°C umsetzt ("Meerwein-Arylierung" ; vgl. hierzu auch Organic Reactions 11, 189 [1960] ; Organic Reactions 24, 225 [1976] oder C. Ferri "Reaktionen der organischen Synthese" S. 319, Thieme Verlag Stuttgart 1978) und dann in einer 2. Stufe die so erhältlichen substituierten α-Chlor-β-phenylpropionitrile der Formel (V),

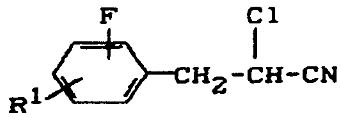 (V)

in welcher

$R^1$ die oben angegebene Bedeutung hat,

mit Basen, wie beispielsweise Triethylamin oder Diazabicycloundecen, in üblicher Art und Weise gegebenenfalls in Gegenwart eines Verdünnungsmittels, wie beispielsweise Tetrahydrofuran, bei Temperaturen zwischen 0°C und 50°C dehydrohalogeniert (vgl. auch die Herstellungsbeispiele) oder alternativ, wenn man

b) substituierte Benzaldehyde der Formel (VI),

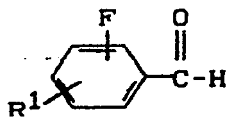 (VI)

in welcher

$R^1$ die oben angegebene Bedeutung hat,

mit Cyanessigsäure der Formel (VII)

$$NC\text{-}CH_2\text{-}COOH \quad (VII)$$

in üblicher Art und Weise in Gegenwart einer Base, wie beispielsweise Piperidin oder Pyridin, und gegebenenfalls in Gegenwart eines geeigneten Verdünnungsmittels, wie beispielsweise Pyridin, bei Temperaturen zwischen 50°C und 120°C kondensiert und gleichzeitig decarboxyliert (vgl. z.B. "Organikum" S. 571/572 ; 15. Auflage ; VEB Deutscher Verlag der Wissenschaften Berlin 1981 sowie die Herstellungsbeispiele), und die so [nach Verfahren (a) oder (b)] erhältlichen substituierten Zimtsäurenitrile der Formel (VIII),

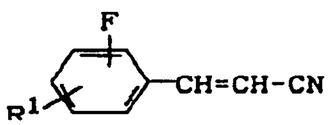 (VIII)

in welcher

$R^1$ die oben angegebene Bedeutung hat,

mit Sulfonylmethylisocyaniden der Formel (IX),

$$R^3\text{-}SO_2\text{-}CH_2\text{-}NC \quad (IX)$$

in welcher

$R^3$ für Alkyl oder für gegebenenfalls substituiertes Aryl, insbesondere für Methyl, für 4-Methylphenyl, 4-Chlorphenyl oder für Phenyl steht,

in Gegenwart einer Base wie beispielsweise Natriumhydrid und gegebenenfalls in Gegenwart eines Verdünnungsmittels wie beispielsweise Tetrahydrofuran bei Temperaturen zwischen −20°C und +50°C umsetzt.

Fluoraniline der Formel (IV) sind teilweise bekannt (vgl. z.B. J. org. Chem. 39, 1758-1761 [1974] : J. med. Chem. 12, 195-196 [1969] oder US-PS 3900519).

Die weiterhin als Vorprodukte zur Herstellung der neuen Ausgangsprodukte der Formel (II) nach Variante b) benötigten Fluorbenzaldehyde der Formel (VI) sind größtenteils bekannt (vgl. z.B. Chem. Abstr. 100 : 209 388k bzw. Jap. Pat. 58/222045), so ist der 3-Chlor-5-fluor-benzaldehyd mit Reg.-No. 90390-49-1 und der 3-Chlor-6-fluor-benzaldehyd mit Reg.-No. 96515-79-6 in Chem. Abs. registriert. Die Cyanessigsäure der Formel (VII) ist eine allgemein bekannte Verbindung der organischen Chemie.

Die weiterhin als Vorprodukte zur Herstellung der neuen Ausgangsprodukte der Formel (II) benötigten Sulfonyl-methylisocyanide der Formel (IX) sind ebenfalls bekannt (vgl. z.B., Synthesis 1985, 400-402 ; Org. Syntheses 57, 102-106 [1977] ; J. org. Chem. 42, 1153-1159 [1977] ; Tetrahedron Lett. 1972, 2367-2368).

Die zur Durchführung des erfindungsgemäßen Verfahrens weiterhin als Ausgangsstoffe benötigten Acylierungsmittel sind durch die Formel (III) allgemein definiert. In dieser Formel (III) steht R vorzugsweise für diejenigen Reste, die bereits im Zusammenhang mit der Beschreibung der erfindungsgemäßen Stoffe der Formel (I) als bevorzugt für diesen Substituenten genannt wurden.

E steht vorzugsweise für Halogen, insbesondere für Chlor oder Brom, oder für einen Anhydridrest der Formel

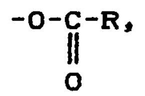

wobei R die oben angegebene Bedeutung hat.

Die Acylierungsmittel der Formel (III) sind allgemein bekannte Verbindungen der organischen Chemie.

Alternativ zur Herstellung der Vorprodukte der Formel (II) mit Hilfe der im vorangegangenen beschriebenen Herstellungsverfahren sind verschiedene weitere Herstellungsverfahren zur Herstellung der Vorprodukte der Formel (II) denkbar.

So erhält man 1H-3-Cyano-4-phenyl-pyrrole der Formel (II) beispielsweise auch, wenn man α-Cyanozimtsäureester in Gegenwart von Basen und in Gegenwart von Kupfer- (II)-Salzen mit p-Toluol-sulfonylmethylisocyanid umsetzt (vgl. J6-1030-571 oder J6-1200-984) oder wenn man α-substituierte Zimtsäurenitrile in Gegenwart von Natriumhydrid mit Isocyanoessigsäureestern cyclisiert, die so erhältlichen Pyrrol-2-carbonsäureester mit Basen verseift und anschließend thermisch decarboxyliert (vgl. JP 59/212468) oder wenn man Phenacylaminderivate mit geeignet substituierten Acrylnitril-Derivaten umsetzt (vgl. EP 174910) oder wenn man 3-Cyano-4-phenyl-$\Delta^2$-pyrroline in Gegenwart von Kupfer-II-Salzen oder Eisen-III-salzen oxidiert (vgl. EP 183217) oder wenn man α-Cyanoacrylsäurederivate mit Isocyanoessigsäureestern in Gegenwart einer Base umsetzt und die so erhältlichen $\Delta^2$-Pyrrolin-2-carbonsäurederivate in einer 2. Stufe in Gegenwart einer Base und in Gegenwart eines Metallsalzkatalysators oxidativ decarboxyliert (vgl. Deutsche Patentanmeldung P 3718375 vom 02.06.1987).

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren wird vorzugsweise in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetall-carbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen −20°C und 150°C, vorzugsweise bei Temperaturen zwischen 0°C und 80°C.

Zur Durchführung des erfindungsgemäßen Verfahrens setzt man pro Mol an 1H-3-Cyano-4-phenyl-pyrrol

der Formel (II) im allgemeinen 1.0 bis 5.0 Mol, vorzugsweise 1.0 bis 1.5 Mol an Acylierungsmittel der Formel (III) und gegebenenfalls 1.0 bis 5.0 Mol, vorzugsweise 1.0 bis 1.5 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind z.B. für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt :
Pythium-Arten, wie beispielsweise Pythium ultimum ;
Phytophthora-Arten, wie beispielsweise Phytophthora infestans ;
Pseudoperonospora-Arten, wie beispielsweise Pseudoperonospora humuli oder Pseudoperonospora cubensis ;
Plasmopara-Arten, wie beispielsweise Plasmopara viticola ;
Peronospora-Arten, wie beispielsweise Peronospora pisi oder P. brassicae ;
Erysiphe-Arten, wie beispielsweise Erysiphe graminis ;
Sphaerotheca-Arten, wie beispielsweise Sphaerotheca fuliginea ;
Podosphaera-Arten, wie beispielsweise Podosphaera leucotricha ;
Venturia-Arten, wie beispielsweise Venturia inaequalis ;
Pyrenophora-Arten, wie beispielsweise Pyrenophora teres oder P. graminea
(Konidienform : Drechslera, Syn : Helminthosporium) ;
Cochliobolus-Arten, wie beispielsweise Cochliobolus sativus
(Konidienform : Drechslera, Syn : Helminthosporium) ;
Uromyces-Arten, wie beispielsweise Uromyces appendiculatus ;
Puccinia-Arten, wie beispielsweise Puccinia recondita ;
Tilletia-Arten, wie beispielsweise Tilletia caries ;
Ustilago-Arten, wie beispielsweise Ustilago nuda oder Ustilago avenae ;
Pellicularia-Arten, wie beispielsweise Pellicularia sasakii ;
Pyricularia-Arten, wie beispielsweise Pyricularia oryzae ;
Fusarium-Arten, wie beispielsweise Fusarium culmorum ;
Botrytis-Arten, wie beispielsweise Botrytis cinerea ;
Septoria-Arten, wie beispielsweise Septoria nodorum ;
Leptosphaeria-Arten, wie beispielsweise Leptosphaeria nodorum ;
Cercospora-Arten, wie beispielsweise Cercospora canescens ;
Alternaria-Arten, wie beispielsweise Alternaria brassicae ;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Obst- und Gemüseanbau, wie beispielsweise gegen den Erreger des Bohnengrauschimmels (Botrytis cinerea), oder zur Bekämpfung von Reiskrankheiten, wie beispielsweise gegen den Erreger der Reisfleckenkrankheit (Pyricularia oryzae) oder zur Bekämpfung von Getreidekrankheiten, wie beispielsweise gegen den Erreger der Braunspelzigkeit des Weizens (Leptosphaeria nodorum) oder gegen den Erreger des Getreideschneeschimmels (Fusarium nivale) einsetzen. Darüberhinaus besitzen die erfindungsgemäßen Wirkstoffe eine gute fungizide in-vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage : z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02% am Wirkungsort erforderlich.

Herstellungsbeispiele

Beispiel 1

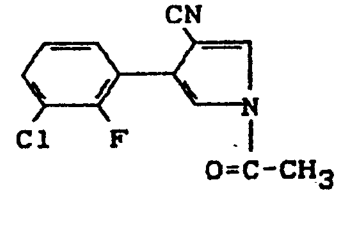

Zu 3,0 g (0.136 Mol) 3-Cyano-4-(2-fluor-3-chlorphenyl)-pyrrol in einer Mischung aus 120 ml Dichlormethan/Tetrahydrofuran (5 : 1) gibt man 1,46 g (0.0143 Mol) Acetanhydrid, 1,45 g (0.0143 Mol) Triethylamin und 0,2 g 4-Dimethylaminopyridin, rührt 16 Stunden bei Raumtemperatur, wäscht zweimal mit Wasser, trocknet über Natriumsulfat und engt im Vakuum ein.

Man erhält 2,4 g (67,2% der Theorie) an 1-Acetyl-3-cyano-4-(2-fluor-3-chlorphenyl)-pyrrol vom Schmelzpunkt 141°C-142°C.

Beispiel 2

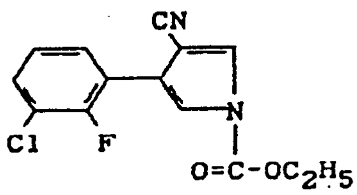

Zu 0,65 g (0.022 Mol) Natriumhydrid (80 prozentig in Paraffinöl) in 20 ml Dimethoxyethan gibt man bei Raumtemperatur tropfenweise unter Rühren 4,0 g (0.0182 Mol) 3-Cyano-4-(2-fluor-3-chlorphenyl)-pyrrol in 60 ml Dimethoxyethan und rührt anschließend zwei weitere Stunden bei Raumtemperatur. Danach gibt man ebenfalls bei Raumtemperatur tropfenweise unter Rühren 3,4 g (0.032 Mol) Chlorameisensäureethylester in 20 ml Dimethoxyethan gelöst zu, rührt weitere 3 Stunden bei Raumtemperatur, hydrolysiert mit Wasser, extrahiert mit Essigester, wäscht zweimal mit Wasser, trocknet über Natriumsulfat und entfernt das Lösungsmittel im Vakuum.

Man erhält 4,9 g (92% der Theorie) an 1-Ethoxycarbonyl-3-cyano-4-(2-fluor-3-chlorphenyl)-pyrrol vom Schmelzpunkt 149°C.

Herstellung der Ausgangsverbindung

Beispiel II-1

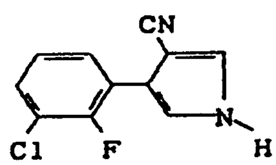

Zu 1,4 g (0.0464 Mol) Natriumydrid (80 %ig in Mineralöl) in 17,5 ml Tetrahydrofuran unter einer Argonschutzgasatmosphäre gibt man bei −10°C bis −20°C tropfenweise unter Rühren eine Lösung von 6,0 g (0.0331 Mol) 3-(2-Fluor-3-chlorphenyl)-acrylnitril und 7,8 g (0.0431 Mol) p-Toluolsulfonylmethylisocyanid in 20 ml einer Mischung aus Tetrahydrofuran/Dimethylsulfoxid (5 : 1). Nach beendeter Zugabe läßt man die Reaktionsmischung auf Raumtemperatur kommen, gibt Wasser zu, extrahiert mehrfach mit Essigester, wäscht die vereinigten Essigesterphasen mit Wasser, trocknet über Natriumsulfat und engt im Vakuum ein. Der Rückstand wird durch Chromatographie an Kieselgel (Laufmittel : Cyclohexan/Essigester 5 : 1) gereinigt.

Man erhält 3,3 g (45% der Theorie) an 3-Cyano-4-(2-fluor-3-chlorphenyl)-pyrrol vom Schmelzpunkt 180°C-181°C.

Beispiel VIII-1

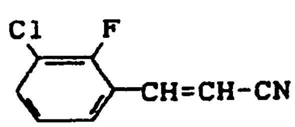

Zu einer Lösung aus 40,1 g (0.25 Mol) 2-Fluor-3-chlorbenzaldehyd (vgl. z.B. DE 3129274) in 170 ml Pyridin gibt man 2,5 ml Piperidin und 22,9 g (0.27 Mol) Cyanessigsäure und erhitzt 14 Stunden auf Rückflußtemperatur. Zur Aufarbeitung engt man im Vakuum ein, nimmt den Rückstand in Essigester auf, wäscht nacheinander mit 1 normaler Salzsäure, mit wässriger Natriumhydrogensulfitlösung sowie mit Wasser, trocknet über Natriumsulfat und engt im Vakuum ein. Das verbleibende Öl läßt sich durch Chromtographie an Kieselgel (Laufmittel: Cyclohexan Essigester 5 : 1) reinigen.

Man erhält 16,9 g (37% der Theorie) an 3-(2-Fluor-3-chlorphenyl)-acrylnitril vom Schmelzpunkt 90°C-92°C.

In entsprechender Weise und gemäß den allgemeinen Angaben zur Herstellung erhält man die folgenden 3-Cyano-4-phenyl-pyrrol-Derivate der allgemeinen Formel (I) :

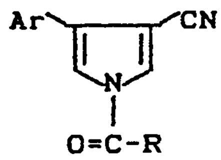

(I)

| Bsp. Nr. | Ar | R | Schmelzpunkt [°C] |
|---|---|---|---|
| 3 | 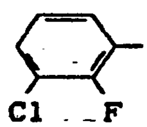 | $-CH_2-OCH_3$ | 119-120 |

## Anwendungsbeispiele

In den folgenden Anwendungsbeispielen wurden die nachstehend aufgeführten Verbindungen als Vergleichsubstanzen eingesetzt ;

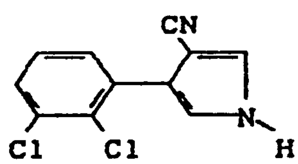

(A)

3-Cyano-4-(2,3-dichlorphenyl)-pyrrol (vgl. EP 174910 sowie EP 236272)
und/oder

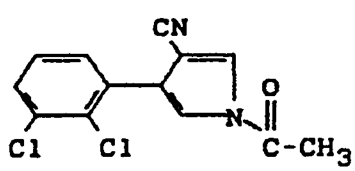

(B)

1-Acetyl-3-cyano-4-(2,3-dichlorphenyl)-pyrrol (vgl. DE-OS 2927480)
und/oder

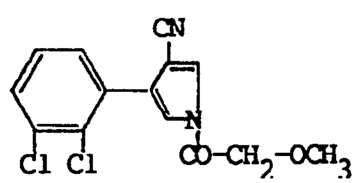

1-Methoxyacetyl-3-cyano-4-(2,3-dichlorphenyl)-pyrrol (vgl. Jp. Kokai Tokkyo Koho 81-79672).

### Beispiel A

Botrytis-Test (Bohne)/protektiv

Lösungsmittel : 4,7 Gewichtsteile Aceton

Emulgator : D,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt daß Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigen in diesem Test z.B. die Verbindungen gemäß den Herstellungsbeispielen : 1 und 3.

Beispiel B

Pyrenophora teres-Test (Gerste)/protektiv

Lösungsmittel : 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20°C und 100% relativer Luftfeuchtigkeit in einer Inkubationskabine.

Die Pflanzen werden in einem Gewächshaus bei einer Temperatur von ca. 20°C und einer relativen Luftfeuchtigkeit von ca. 80% aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen : 1 und 3.

## Patentansprüche

1. 3-Cyano-4-phenyl-pyrrole der allgemeinen Formel (I),

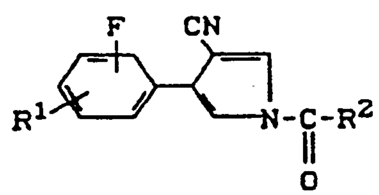

(I)

in welcher

$R^1$ für Chlor steht und
$R^2$ für jeweils geradkettiges oder verzweigtes Alkyl, Alkoxy oder Alkoxyalkyl mit jeweils 1 bis 6 Kohlenstoffatomen in den einzelnen Alkylteilen steht.

2. 3-Cyano-4-phenyl-pyrrole gemäß Anspruch 1, wobei in der Formel (I)

$R^1$ für Chlor steht und
$R^2$ für jeweils geradkettiges oder verzweigtes Alkyl, Alkoxy oder Alkoxyalkyl mit jeweils 1 bis 4 Kohlenstoffatomen in den einzelnen Alkylteilen steht.

3. 3-Cyano-4-phenyl-pyrrole gemäß Anspruch 1, wobei in der Formel (I)

$R^1$ für Chlor steht und
$R^2$ für Methyl, Ethyl, Methoxy, Ethoxy, Methoxymethyl oder Ethoxymethyl steht.

4. Verfahren zur Herstellung von 3-Cyano-4-phenyl-pyrrolen der allgemeinen Formel (I),

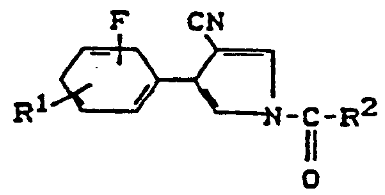

(I)

in welcher

$R^1$ für Chlor steht und
$R^2$ für jeweils geradkettiges oder verzweigtes Alkyl, Alkoxy oder Alkoxyalkyl mit jeweils 1 bis 6 Kohlenstoffatomen in den einzelnen Alkylteilen steht, dadurch gekennzeichnet, daß man 1H-3-Cyano-4-phenyl-pyrrole der Formel (II),

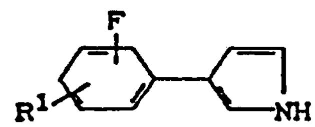

(II)

in welcher

$R^1$ die oben angegebene Bedeutung hat,

mit Acylierungsmitteln der Formel (III)

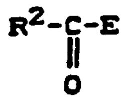

(III)

in welcher

$R^2$ die oben angegebene Bedeutung hat und
E für eine elektronenanziehende Abgangsgruppe steht, gegebenenfalls in Gegenwart eines Verdünnungsmittels und gegebenenfalls in Gegenwart eines Reaktionshilfsmittels umsetzt.

5. Schädlingsbekämpfungsmittel, gekennzeichnet durch einen Gehalt an mindestens einem 3-Cyano-4-phenyl-pyrrol der Formel (I) nach den Ansprüchen 1 und 5.

6. Verwendung von 3-Cyano-4-phenyl-pyrrolen der Formel (I) nach Ansprüchen 1 und 4 zur Bekämpfung von Schädlingen.

7. Verfahren zur Bekämpfung von Schädlingen, dadurch gekennzeichnet, daß man 3-Cyano-4-phenyl-pyrrole der Formel (I) nach den Ansprüchen 1 und 4 auf Schädlinge und/oder ihren Lebensraum einwirken läßt.

8. Verfahren zur Herstellung von Schädlingsbekäpfungsmitteln, dadurch gekennzeichnet, daß man 3-Cyano-4-phenyl-pyrrole der Formel (I) nach den Asprüchen 1 und 4 mit Streckmitteln und/oder oberflächenaktiven Mitteln vermischt.

## Claims

1. 3-Cyano-4-phenyl-pyrroles of the general formula (I)

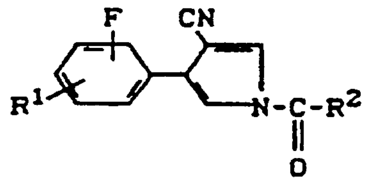

(I)

in which

$R^1$ represents chlorine and

$R^2$ represents in each case straight-chain or branched alkyl, alkoxy or alkoxyalkyl, each having 1 to 6 carbon atoms in the individual alkyl moieties.

2. 3-Cyano-4-phenyl-pyrroles according to Claim 1, where in the formula (I)

$R^1$ represents chlorine and

$R^2$ represents in each case straight-chain or branched alkyl, alkoxy or alkoxyalkyl, each having 1 to 4 carbon atoms in the individual alkyl moieties.

3. 3-Cyano-4-phenyl-pyrroles according to Claim 1, where in the formula (I)

$R^1$ represents chlorine and

$R^2$ represents methyl, ethyl, methoxy, ethoxy, methoxymethyl or ethoxymethyl.

4. Process for the preparation of 3-cyano-4-phenyl-pyrroles of the general formula (I)

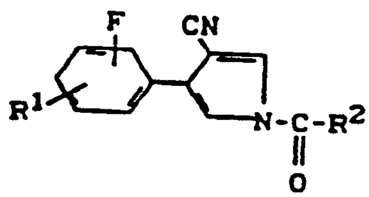

(I)

in which

$R^1$ represents chlorine and

$R^2$ represents in each case straight-chain or branched alkyl, alkoxy or alkoxyalkyl, each having 1 to 6 carbon atoms in the individual alkyl moieties, characterised in that 1H-3-cyano-4-phenyl-pyrroles of the formula (II)

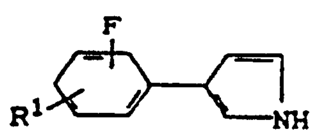

(II)

in which

$R^1$ has the abovementioned meaning

are reacted with acylating agents of the formula (III)

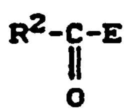

(III)

in which

$R^2$ has the abovementioned meaning and

E represents an electron-attracting leaving group, if appropriate in the presence of a diluent and if appropriate in the presence of a reaction auxiliary.

5. Pesticides, characterised in that they contain at least one 3-cyano-4-phenyl-pyrrole of the formula (I) according to Claims 1 and 4.

6. Use of 3-cyano-4-phenyl-pyrroles of the formula (I) according to Claims 1 and 4 for combating pests.

7. Method of combating pests, characterised in that 3-cyano-4-phenyl-pyrroles of the formula (I) according to Claims 1 and 4 are allowed to act on pests and/or their environment.

8. Process for the preparation of pesticides, characterised in that 3-cyano-4-phenyl-pyrroles of the formula (I) according to Claims 1 and 4 are mixed with extenders and/or surface-active agents.

**Revendications**

1. 3-cyano-4-phényl-pyrroles de formule générale (I)

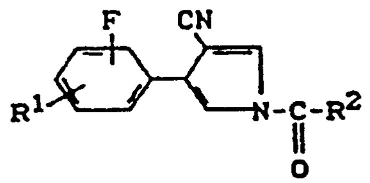

(I)

dans laquelle

$R^1$ représente le chlore et

$R^2$ représente alkyle, alcoxy ou alcoxyalkyle, chaque fois à chaîne droite ou ramifiée, ayant chaque fois 1 à 6 atomes de carbone dans les parties alkyles séparées.

2. 3-cyano-4-phényl-pyrroles selon la revendication 1, dans lesquels, dans la formule (I),

$R^1$ représente le chlore et

$R^2$ représente alkyle, alcoxy ou alcoxyalkyle chaque fois à chaîne droite ou ramifiée ayant chaque fois 1 à 4 atomes de carbone dans les parties alkyles séparées.

3. 3-cyano-4-phényl-pyrroles selon la revendication 1, dans lesquels, dans la formule (I),

$R^1$ représente le chlore et

$R^2$ représente méthyle, éthyle, méthoxy, éthoxy, méthoxyméthyle ou éthoxyméthyle.

4. Procédé pour la préparation de 3-cyano-4-phénylpyrroles de formule générale (I)

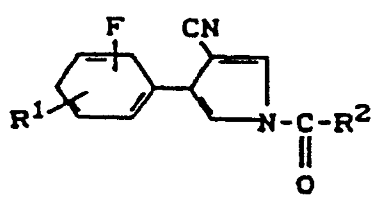

(I)

dans laquelle

$R^1$ représente le chlore et

$R^2$ représente alkyle, alcoxy ou alcoxyalkyle, chaque fois à chaîne droite ou ramifiée, ayant chaque fois 1 à 6 atomes de carbone dans les parties alkyles séparées, caractérisé en ce que l'on fait réagir des 1H-3-cyano-4-phénylpyrroles de formule (II)

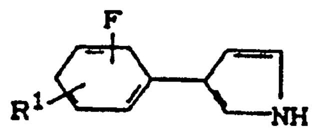 (II)

dans laquelle

$R^1$ a la signification indiquée ci-dessus,
avec des agents d'acylation de formule (III)

$$R^2-\underset{\underset{O}{\|}}{C}-E \qquad (III)$$

dans laquelle

$R^2$ a la signification indiquée ci-dessus et
E représente un groupe éliminable attirant les électrons, éventuellement en présence d'un agent diluant et éventuellement en présence d'un agent auxiliaire de réaction.

5. Agents de lutte contre les parasites, caractérisés en ce qu'ils contiennent au moins un 3-cyano-4-phényl-pyrrole de formule (I) selon les revendications 1 et 5.

6. Utilisation de 3-cyano-4-phényl-pyrroles de formule (I) selon les revendications 1 et 4 pour la lutte contre les parasites.

7. Procédé pour la lutte contre les parasites, caractérisé en ce que l'on fait agir sur les parasites et/ou leur espace vital des 3-cyano-4-phényl-pyrroles de formule (I) selon les revendications 1 et 4.

8. Procédé pour la préparation d'agents de lutte contre les parasites, caractérisé en ce que l'on mélange des 3-cyano-4-phényl-pyrroles de formule (I) selon les revendications 1 et 4 avec des agents diluants et/ou des agents tensioactifs.